# EUROPÄISCHE PATENTSCHRIFT

(11) **EP 0 569 843 B1**
(45) Veröffentlichungstag und Bekanntmachung des Hinweises auf die Patenterteilung: **15.11.1995**
(21) Anmeldenummer: 93107306.8
(22) Anmeldetag: 04.05.1993
(51) Int. Cl.: C11D 1/825, C11D 17/00, A61K 7/08, A61K 7/48, A61K 7/06

(54) **Nichtionische, fliessfähige Perlglanzdispersionen**
Non-ionic free-flowing pearl lustre dispersions
Dispersions nacrés nonioniques capables d'écouler

(30) Priorität: 13.05.1992 DE 4215732
(43) Veröffentlichungstag der Anmeldung: 18.11.1993
(73) Patentinhaber: HOECHST AKTIENGESELLSCHAFT, 65926 Frankfurt am Main (DE)
(72) Erfinder: Reng, Alwin, W-6233 Kelkheim (Taunus) (DE); Skrypzak, Werner, W-6237 Liederbach (DE); Kunz, Walter, W-6234 Hattersheim/Main (DE)

(56) Entgegenhaltungen:
- EP-A- 0 158 174
- EP-A- 0 535 693
- WO-A-92/13512
- PATENT ABSTRACTS OF JAPAN vol. 9, no. 166 (C-290)11. Juli 1985

## Beschreibung

Bei der Herstellung von kosmetischen Haar- und Körperreinigungsmitteln, Geschirrspülmitteln und flüssigen Wasch- und Reinigungsmitteln werden zur Verbesserung des optischen Aspektes, und damit zur Steigerung des Handelswertes, häufig Substanzen verwendet, die den genannten Präparaten ein perlglanzartiges Aussehen verleihen. Um einen solchen Perlglanz- bzw. Seidenglanz-Effekt zu erreichen, sind verschiedene Substanzen bekannt, beispielsweise pulverförmige Naturstoffe, wie Glimmer, Fischsilber, anorganische Materialien, wie Wismutoxichlorid und Titandioxidpigmente, ferner Metallsalze höherer Fettsäuren, Fettsäureglykolester und Fettsäurealkanolamide im Gemisch mit anderen Tensiden.

Üblicherweise wird zu diesem Zweck ein Fettsäureglykolester in Kombination mit einem Fettsäurealkanolamid als perlglanzgebende Komponente verwendet. Diese festen Substanzen werden in traditioneller Weise bei der Herstellung von kosmetischen und technischen Wasch- und Reinigungsmitteln mit Perlglanzeffekt durch Erwärmen des Ansatzes über den Schmelzpunkt der erwähnten Substanzen in der Tensidphase dispergiert. Beim Abkühlen fallen diese perlglanzgebenden Komponenten als Kristalle aus und verleihen den Fertigprodukten den gewünschten Perlglanzeffekt.

Aus US-A-4 620 976 sind fließfähige Perlglanzdispersionen bekannt, die im wesentlichen aus Fettsäureglykolester, Fettsäurealkanolamiden, ionischen Tensiden, ein- oder zweiwertigen Metallsalzen und Wasser bestehen.

Die US-A-4 654 163 beschreibt nichtionische, fließfähige Perglanzdispersionen aus Fettsäureglykolester, Fettsäurealkanolamiden, nichtionischen Tensiden und Wasser.

JP-A-60 038 309 offenbart eine Zusammensetzung mit Perlglanz, welche eine oberflächenaktive Substanz, einen Fettsäureglykoldiester und eine Polyetherverbindung mit einem mehrwertigen Alkohol als Endgruppe enthält.

Es hat sich gezeigt, daß bei der Verwendung von Fettsäurealkanolamiden auch aufgrund vorhandener Restmengen an sekundären Aminen, gesundheitsgefährdende Nitrosamine gebildet werden können. Es wurde auch gefunden, daß es bei dem Einsatz der üblichen Fettsäurealkanolamide, wie Kokosfettsäuremonoethanolamid, in der Perlglanzdispersion zur Bildung von relativ großen Kristallen kommt, die zu einer verstärkten Separation neigen und andererseits eine niedrige optische Dichte, d.h. eine geringe Ausgiebigkeit bewirken.

Aufgabe der Erfindung ist es Perlglanzdispersionen zur Verfügung zu stellen, die frei von Fettsäurealkanolamiden sind und dennoch die Eigenschaften der bekannten Perlglanzdispersionen, wie niedrige Viskosität, gute Lagerstabilität und ausgezeichneten Perlglanzeffekt besitzen.

Gegenstand der Erfindung sind nichtionische, fließfähige Perlglanzdispersionen, bestehend aus
5 - 30 Gew.-% eines Fettsäureglykolesters der allgemeinen Formel I

R¹ - C(O) - (O-A)ₘ - O - X (I)

wobei R¹ eine gesättigte oder ungesättigte Kohlenwasserstoffkette mit 13 - 21 Kohlenstoffatomen,
A eine Gruppe der Formel -C₂H₄- oder -C₃H₆-, vorzugsweise -C₂H₄-,
X ein Wasserstoffatom oder eine Gruppe der allgemeinen Formel

R⁵ - C(O)-

R⁵ eine gesättigte oder ungesättigte Kohlenwasserstoffkette mit 13 - 21 Kohlenstoffatomen,
R¹ und R⁵ unabhängig voneinander gleich oder verschieden, bevorzugt gleich, sind und m eine Zahl von 1 - 10, vorzugsweise 1 - 3 bedeuten,
0,1 - 20 Gew.-% eines nichtionischen Tensides der allgemeinen Formel II

R² - O - (A-O)ₙ - (B-O)ₚ - R³ (II)

wobei R² eine gesättigte oder ungesättigte Kohlenwasserstoffgruppe mit 8 - 30 Kohlenstoffatomen,
R³ ein Wasserstoffatom oder eine Gruppe der allgemeinen Formel

- CH₂ - O - R⁴,

R⁴ eine gesättigte oder ungesättigte Kohlenwasserstoffgruppe mit 1 - 10 Kohlenstoffatomen,
A und B unabhängig voneinander eine Gruppe der Formel -C₂H₄- oder -C₃H₆- und
n und p jeweils für sich eine Zahl von 1 - 30, vorzugsweise 3 - 10 bedeuten,
sowie Wasser in der an 100 Gew.-% fehlenden Menge.

Die zur Herstellung der erfindungsgemäßen Perlglanzdispersionen benötigten Komponenten sowie die Einsatzkonzentration werden nachfolgend beschrieben.

Als Perlglanzbildner haben sich Fettsäureglykolester der allgemeinen Formel I sowie Mischungen dieser Substanzen bewährt. Die günstigsten Eigenschaften zeigen Verbindungen, in denen R¹ ein Alkylrest mit 15 - 17 Kohlenstoffatomen,
m = 1 und X ein Rest der Formel R⁵-C(O)- ist, wobei R⁵ ein Alkylrest mit 15 - 17 Kohlenstoffatomen ist und R¹ und R⁵ gleich sind.
Die Einsatzkonzentration dieser Fettsäureglykolester beträgt vorzugsweise 15 bis 30 Gew.-%, wobei die optimale Konzentration bei 20 bis 25 Gew.-%, bezogen auf das Gewicht der Mischung liegt.

Unter den genannten nichtionischen Tensiden entsprechend der allgemeinen Formel II haben sich besonders folgende Verbindungen und Mischungen davon als günstig erwiesen:
Verbindungen bei denen R² eine Alkylgruppe mit 10 bis 18 Kohlenstoffatomen, linear oder verzweigt und A eine Gruppe der Formel -C₂H₄-, n eine Zahl von 5 - 20,
p = 0 und R³ ein Wasserstoffatom ist.
Ebenso Verbindungen, bei denen R² eine Alkylgruppe mit 10 bis 18 Kohlenstoffatomen, linear oder verzweigt, und A und B unabhängig voneinander eine Gruppe der Formel -C₂H₄- oder -C₃H₆- und n und m jeweils eine Zahl von 1 bis 20, vorzugsweise 3 bis 10, bedeuten.

Die Einsatzkonzentration dieser nichtionischen Tenside beträgt vorzugsweise 5 bis 15 Gew.-%, bezogen auf das Gesamtgewicht.

Die erfindungsgemäßen Perlglanzdispersionen können 5 bis 40 Gew.-%, vorzugsweise 20 bis 30 Gew.-%, bezogen auf das Gesamtgewicht der Mischung niedrig molekulare, mehrwertige Alkohole und/oder Polyole enthalten. Beispiele für niedrig-molekulare, mehrwertige Alkohole sind Ethylenglykol, Diethylenglykol, 1,2-Propylenglykol, 1,3-Propylenglykol, Sorbit, Mannit, Xylit und Glycerin. Beispielhaft für Polyole seien Polyethylenglykole mit Molmassen zwischen 200 und 800 genannt. Unter den geeigneten niedrig-molekularen, mehrwertigen Alkoholen und Polyolen haben sich besonders Polyethylenglykole mit den Molmassen zwischen 200 und 600 alleine oder in Kombination mit 1,2-Propylenglykol und/oder Glycerin bewährt.

Im Rahmen der oben genannten Einsatzkonzentrationen der einzelnen Komponenten der Formeln I und II werden unter Einhaltung der nachstehend beschriebenen Herstellbedingungen optimale Perlglanzdispersionen erhalten, die folgende vorteilhafte Eigenschaften aufweisen:
a) ausgezeichnetes Fließverhalten auch bei tieferen Temperaturen (+5°C); hierdurch ist die Möglichkeit einer kontinuierlichen Verarbeitung gegeben;
b) nichtionischer Aufbau, dadurch keine chemische Reaktion mit kationischen Tensiden;
c) frei von nitrosaminbildenden Amin- bzw. Alkanolamiden;
d) kein zwingender Zusatz von Konservierungsstoffen.

Es hat sich gezeigt, daß durch den Zusatz von niedrig-molekularen, mehrwertigen Alkoholen und/oder Polyolen eine bakteriostatische, oder auch bakterizide Wirkung erzielt wird, so daß der bisher übliche Zusatz von Konservierungsmittel nicht zwingend erforderlich ist.

Die restliche Menge zu 100 Gew.-% der Mischung wird mit Wasser ergänzt. Bei dem verwendeten Wasser handelt es sich um entsalztes Wasser.

Neben den vorstehend genannten Bestandteilen Fettsäureglykolester, nichtionischen Tensid, Wasser und gegebenenfalls niedrig-molekularen, mehrwertigen Alkoholen und/oder Polyolen, können die erfindungsgemäßen Perlglanzdispersionen im Bedarfsfall noch Zusätze wie Puffersubstanzen, Elektrolyte und/oder Konservierungsmittel, wie Benzoesäure oder Sorbinsäure enthalten. Die Menge an diesen Zusätzen liegt zwischen 0 und 1,0 Gew.-%, bevorzugt 0,3 bis 0,4 Gew.-%.

Zur Herstellung der erfindungsgemäßen Perlglanzdispersionen wird wie folgt verfahren:
In einem Mischgefäß (heizbarer Kessel) werden die beiden Komponenten der allgemeinen Formeln I und II vorgelegt und unter Erhitzen auf ca. 76°C geschmolzen. In diese Schmelze wird gegebenenfalls unter Rühren eine ebenfalls auf 75°C erhitzte wäßrige Lösung, bzw. eine Mischung mit Polyolen bzw. niedrigmolekularen, mehrwertigen Alkoholen zugegeben. Die Rührgeschwindigkeit beträgt je nach Ansatz und Kesselgröße 10 bis 100 UpM. Die gebildete Dispersion wird unter Rühren bis zu einer Endtemperatur von 20 bis 30°C abgekühlt. Die Geschwindigkeit der Abkühlung erfolgt unter kontrollierten Bedingungen um einen konstanten reproduzierbaren Perlglanzeffekt zu erreichen.
Während des Abkühlvorganges beginnt die Komponente der Formel I bei ca. 50 bis 60°C zu kristallisieren und bildet den gewünschten Perlglanzeffekt.
Der pH-Wert der Dispersionen liegt im Bereich von 4 bis 9, vorzugsweise im Bereich zwischen 5 bis 8.

Die erfindungsgemäßen Perlglanzdispersionen können bei Raumtemperatur flüssigen Haar- und Körperreinigungsmitteln, flüssigen Geschirrspülmitteln, sowie flüssigen Wasch- und Reinigungsmitteln zugesetzt werden. Hierdurch werden Endprodukte erhalten, die einen ausgezeichneten Perlglanz aufweisen. Die hierzu benötigte Menge der Perlglanzdispersion liegt zwischen 1 und 15, bevorzugt 2 bis 5 Gew.-%. Da die erfindungsgemäße Perlglanzdispersion bei Temperaturen über 5°C eine vergleichsweise niedrige Viskosität aufweist, besteht die Möglichkeit, die Dispersion mit Hilfe von automatischen Pump-, Dosier- und Mischanlagen zu verarbeiten. Von besonderem Interesse ist dies bei der vollkontinuierlichen Herstellung von perlglanzhaltigen Fertigprodukten.

Die Herstellung der erfindungsgemäßen Perlglanzdispersion wird an folgenden Beispielen erläutert. Die Mengenangaben beziehen sich jeweils auf Gewichtsprozente. "EO" bedeutet Ethylenoxid und "PO" Propylenoxid.

### Herstellungsbeispiele

### Beispiel 1

| | |
|---|---|
| Monoethylenglykoldistearat | 23,0 % |
| C_{8/18}-Fettalkohol + 8 EO | 4,0 % |
| C_{8/18}-Fettalkohol + 5 EO | 4,0 % |
| C_{12/15}-Fettalkohol + 4 EO + 4 PO | 2,0 % |
| Wasser, Konservierungsmittel (Sorbinsäure) ad | 100,0 % |

### Beispiel 2

| | |
|---|---|
| Monoethylenglykoldistearat | 18,0 % |
| C_{8/18}-Fettalkohol + 8 EO | 5,0 % |
| C_{8/18}-Fettalkohol + 5 EO | 3,0 % |
| C_{12/15}-Fettalkohol + 6 EO + 4 PO | 2,0 % |
| Glycerin | 24,0 % |
| Wasser ad | 100,0% |

### Beispiel 3

| | |
|---|---|
| Ethylenglykolmonostearat | 20,0 % |
| C_{10/12}-Fettalkohol + 4 EO + 4 PO | 2,0 % |
| C_{8/18}-Fettalkohol + 6 EO | 9,0 % |
| Wasser, Konservierungsmittel (Benzoesäure) ad | 100,0 % |

### Beispiel 4

| | |
|---|---|
| Triethylenglykoldistearat | 15,0 % |
| C_{12/15}-Fettalkohol + 6 EO + 4 PO | 3,0 % |
| C_{8/18}-Fettalkohol + 6 EO | 7,0 % |
| Glycerin | 15,0 % |
| Polyethylenglykol 400 | 10,0 % |
| Wasser ad | 100,0 % |

Diese Perlglanzdispersionen, gemäß den Beispielen 1 - 4, wurden nach folgenden Kriterien geprüft:
1. Beurteilung des Perlglanzeffektes
2. Bestimmung der "optischen Dichte"
3. Viskosität bzw. Fließverhalten
4. Lagerstabilität bei höheren und tieferen Temperaturen als Raumtemperatur
5. Konservierungsbelastungstest

Die Prüfungen wurden im Vergleich mit einem marktüblichen Perlglanzmittel entsprechend der folgenden Zusammensetzung durchgeführt:

| | |
|---|---|
| Monoethylenglykoldisterat | 10,0 % |
| Cocosfettsäuremonoethanolamid | 10,0 % |
| Lauryldiglykolethersulfat-Natriumsalz | 18,0 % |
| Wasser, Konservierungsmittel, Elektrolyte ad | 100,0 % |

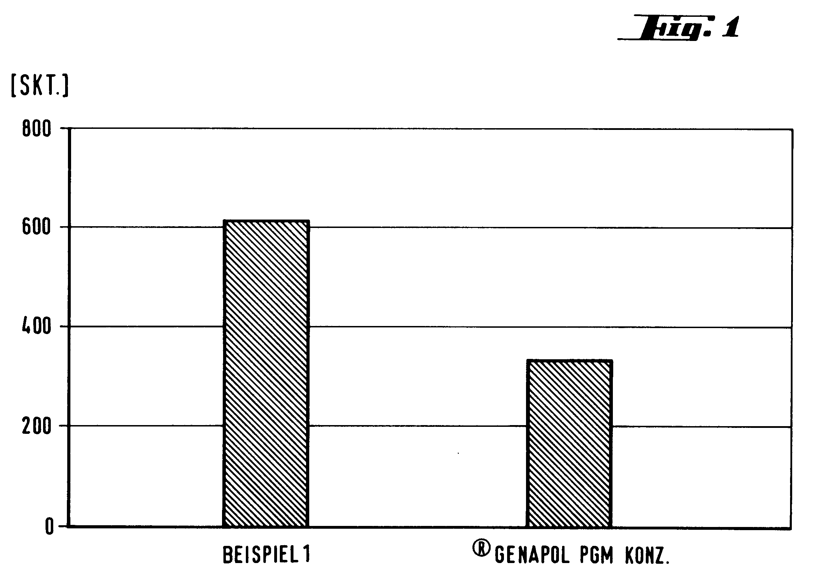

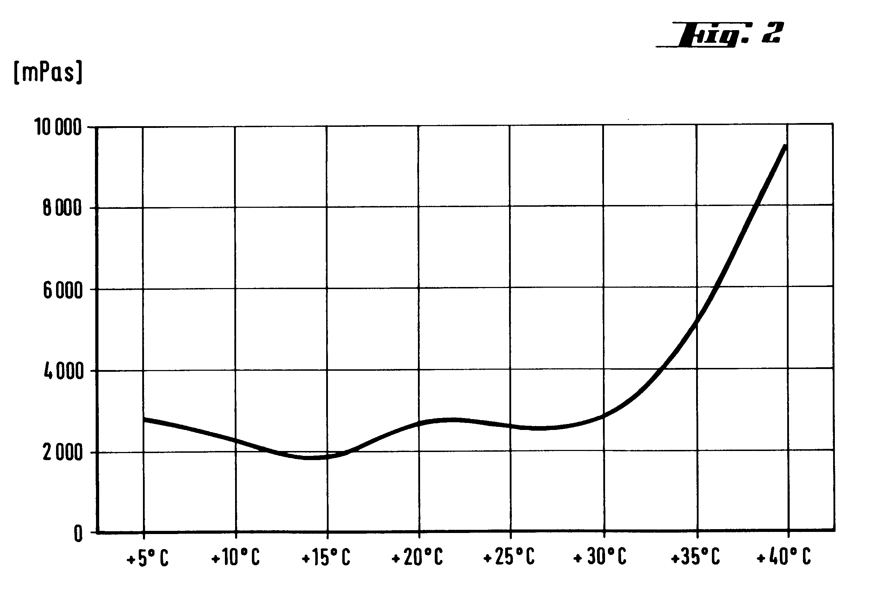

Nachstehend sind die entsprechenden Prüfmethoden und Ergebnisse aufgeführt.
1. Perlglanzeffekt
   Die Beurteilung des Perlglanzeffektes erfolgte visuell nach den Einstufungen matt bis brillant. Es wurden zunächst die Perlglanzkonzentrate entsprechend den Beispielen 1 - 4 direkt beurteilt und anschließend 2 gew.-%ige Verdünnungen in entsalztem Wasser hergestellt. Die erfindungsgemäßen Perlglanzdispersionen besitzen einen ausgeprägten Perlglanzeffekt, der sich in einer hohen Brillanz ausdrückt.
2. Optische Dichte
   Die Prüfung der optischen Dichte, angegeben in Skalenteilen [SKT], erfolgte photometrisch in einer Verdünnung von 4,0 g/l Wasser mit Hilfe eines Trübungsmessgerätes (nach Dr. Lange). Wie die Fig. 1 zeigt, weist das Beispiel 1, im Vergleich zu einem marktüblichen Perlglanzmittel, eine wesentlich höhere optische Dichte und damit eine bessere Ausgiebigkeit beim Einsatz in Fertigprodukten auf.
3. Viskositäts- bzw. Fließverhalten
   Die Viskosität wurde mit einem Brookfield-Viskosimeter, Typ RVT bei 20 UpM bei 20°C, 2,0 gew.-%ige Lösung, bestimmt. In der Fig. 2 sind die Viskositätswerte bei verschiedenen Temperaturen des Perlglanzkonzentrates, entsprechend Beispiel 1, dargestellt. Es ist zu erkennen, daß das Perlglanzkonzentrat bei den praxisüblichen Temperaturen ein sehr günstiges Fließverhalten besitzt, d.h. die Pumpbarkeit und einfache Verarbeitung bei diesen Temperaturen ist gewährleistet.
4. Lagerstabilität bei höheren und tieferen Temperaturen
   Die genannten Perlglanzkonzentrate, entsprechend den Beispielen 1 - 4, wurden drei Monate bei -5°C und sechs Monate bei +40°C gelagert. Nach Beendigung der Lagerversuche konnten keine Anzeichen von Instabilität, d.h. Sedimentationen, festgestellt werden.
5. Konservierungsbelastung
   Das Perlglanzkonzentrat, entsprechend dem Beispiel 2, wurde einem Konservierungsbelastungstest unterzogen. Das Konzentrat besitzt eine bakterizide bzw. bakteriostatische Wirkung, d.h. es wurde während des Prüfzeitraums keine Vermehrung der Mikroorganismen unter den angegebenen Prüfbedingungen beobachtet.

### Anwendungsbeispiele

| 1. Haarshampoo | Gew.-% |
|---|---|
| Kokosemidopropylbetain 30 %ig in Wasser | 18,0 |
| Acylaminopolyglykolethersulfat-Magnesiumsalz, 30 %ig in Wasser | 20,0 |
| Laurylalkohol + 3 EO Perlglanzkonzentrat gemäß Bsp. 1 | 1,0 |
| Wasser ad | 100,0 |

| 2. Duschbad | Gew.-% |
|---|---|
| Lauryldiglykolethersulfat-Natriumsalz, 28 %ig in Wasser | 35,0 |
| Kokosfettsäureisethionat-Natriumsalz | 7,0 |
| Natriumchlorid | 2,5 |
| Perlglanzkonzentrat gemäß Bsp. 3 | 3,0 |
| Wasser ad | 100,0 |

| 3. Geschirrspülmittel | Gew.-% |
|---|---|
| Sekundäres Alkansulfonat, 30 %ig | 70,0 |
| Lauryltriglykolethersulfat-Natriumsalz, 28 %ig | 20,0 |
| Laurylalkohol + 3 EO | 3,0 |
| Perlglanzkonzentrat gemäß Bsp. 4 | 2,5 |
| Wasser ad | 100,0 |

| 4. Nichtionisches Haarnachbehandlungsmittel | Gew.-% |
|---|---|
| Perlglanzkonzentrat gemäß Bsp. 1 | 20,0 |
| ®Tylose H 4000 (Celluloseether) | 1,5 |
| Zitronensäure | 0,3 |
| Wasser ad | 100,0 |

## Patentansprüche

1. Nichtionische, fließfähige Perlglanzdispersionen bestehend aus
5 - 30 Gew.-% eines Fettsäureglykolesters der allgemeinen Formel I
R¹ - C(O) - (O-A)ₘ - O - X (I)
wobei R¹ eine gesättigte oder ungesättigte Kohlenwasserstoffkette mit 13 - 21 Kohlenstoffatomen,
A eine Gruppe der Formel -C₂H₄- oder -C₃H₆-, vorzugsweise -C₂H₄-,
X ein Wasserstoffatom oder eine Gruppe der allgemeinen Formel
R⁵ - C(O) -
R⁵ eine gesättigte oder ungesättigte Kohlenwasserstoffkette mit 13 - 21 Kohlenstoffatomen,
R¹ und R⁵ unabhängig voneinander gleich oder verschieden, bevorzugt gleich, sind
und m eine Zahl von 1 - 10, vorzugsweise 1 - 3 bedeuten,
0,1 - 20 Gew.-% eines nichtionischen Tensides der allgemeinen Formel II
R² - O - (A-O)ₙ - (B-O)ₚ - R³ (II)
wobei R² eine gesättigte oder ungesättigte Kohlenwasserstoffgruppe mit 8 - 30 Kohlenstoffatomen,
R³ ein Wasserstoffatom oder eine Gruppe der allgemeinen Formel
-CH₂ - O - R⁴,
R⁴ eine gesättigte oder ungesättigte Kohlenwaserstoffgruppe mit 1 - 10 Kohlenstoffatomen,
A und B unabhängig voneinander eine Gruppe der Formel -C₂H₄- oder -C₃H₆- und
n und p jeweils für sich eine Zahl von 1 - 30, vorzugsweise 3 - 10 bedeuten,
sowie Wasser in der an 100 Gew.-% fehlenden Menge.

2. Perlglanzdispersionen nach Anspruch 1, dadurch gekennzeichnet, daß in der Formel I R¹ C₁₅-C₁₇-Alkyl, X eine Gruppe der Formel R⁵-C(O)- mit R⁵ gleich C₁₅ -C₁₇-Alkyl und m Eins bedeuten, in der Formel II R² C₁₀-C₁₈-Alkyl, R³ 8ein Wasserstoffatom, n 5 bis 20 und p Null bedeuten.

3. Perlglanzdispersionen nach Anspruch 1 oder 2, dadurch gekennzeichnet, daß sie 15 - 30 Gew.-%, vorzugsweise 20 - 25 Gew.-% Verbindungen der Formel I und 5 - 15 Gew.-% Verbindungen der Formel II, bezogen auf das Gesamtgewicht, enthalten.

4. Perlglanzdispersionen nach einem der Ansprüche 1 bis 3, dadurch gekennzeichnet, daß sie 5 bis 40 Gew.-%, vorzugsweise 20 bis 30 Gew.-%, bezogen auf das Gesamtgewicht, niedrig-molekulare, mehrwertige Alkohole und/oder Polyole enthalten.

## Claims

1. A nonionic, flowable pearlescent dispersion comprising
5 - 30% by weight of a fatty acid glycol ester of the formula I
R¹-C(O)-(O-A)ₘ-O-X (I)
in which R¹ is a saturated or unsaturated hydrocarbon chain having 13 - 21 carbon atoms,
A is a group of the formula -C₂H₄- or -C₃H₆-, preferably -C₂H₄-,
X is a hydrogen atom or a group of the formula
R⁵-C(O)-
R⁵ is a saturated or unsaturated hydrocarbon chain having 13 - 21 carbon atoms,
R¹ and R⁵ independently of one another are identical or different, preferably identical,
and m is a number from 1 to 10, preferably 1 to 3,
0.1 - 20% by weight of a nonionic surfactant of the formula II
R²-O-(A-O)ₙ-(B-O)ₚ-R³ (II)
in which R² is a saturated or unsaturated hydrocarbon group having 8 - 30 carbon atoms,
R³ is a hydrogen atom or a group of the formula
-CH₂-O-R⁴
R⁴ is a saturated or unsaturated hydrocarbon group having 1 - 10 carbon atoms,
A and B independently of one another are a group of the formula -C₂H₄- or -C₃H₆- and
n and p each independently are a number from 1 to 30, preferably 3 to 10,
and water in the amount lacking to make 100% by weight.

2. A pearlescent dispersion as claimed in claim 1, wherein, in the formula I, R¹ is C₁₅-C₁₇-alkyl, X is a group of the formula R⁵-C(O)-, where R⁵ is C₁₅-C₁₇-alkyl, and m is one, and in the formula II, R² is C₁₀-C₁₈-alkyl, R³ is a hydrogen atom, n is 5 to 20 and p is zero.

3. A pearlescent dispersion as claimed in claim 1 or 2, which comprises 15 - 30% by weight, preferably 20 - 25% by weight, of compounds of the formula I and 5 - 15% by weight of compounds of the formula II, based on the total weight.

4. A pearlescent dispersion as claimed in one of claims 1 to 3, which comprises 5 to 40% by weight, preferably 20 to 30% by weight, based on the total weight, of low molecular weight polyhydric alcohols and/or polyols.

## Revendications

1. Dispersions d'aspect nacré, fluides, non ioniques, constituées de
5 à 30 % en poids d'un ester glycolique d'acide gras de formule générale I
R¹ - C(O) - (O-A)ₘ - O - X (I),
R¹ représentant une chaîne hydrocarbonée saturée ou insaturée ayant de 13 à 21 atomes de carbone,
A signifiant un groupe de formule -C₂H₄- ou -C₃H₆-, de préférence -C₂H₄-,
X signifiant un atome d'hydrogène ou un groupe de formule générale
R⁵ - C(O)-
R⁵ signifie une chaîne hydrocarbonée saturée ou insaturée ayant de 13 à 21 atomes de carbone,
R¹ et R⁵ étant, indépendamment l'un de l'autre, identiques ou différents, de préférence identiques, et
m signifiant un nombre allant de 1 à 10, de préférence de 1 à 3,
0,1 à 20 % en poids d'un agent de surface non ionique de formule générale II
R² - O - (A-O)ₙ - (B-O)ₚ - R³ (II),
R² signifiant un groupe hydrocarboné saturé ou insaturé ayant de 8 à 30 atomes de carbone,
R³ signifiant un atome d'hydrogène ou un groupe de formule générale
- CH₂ - O - R⁴,
R⁴ signifiant un groupe hydrocarboné saturé ou insaturé ayant de 1 à 10 atomes de carbone,
A et B signifiant, indépendamment l'un de l'autre, un groupe de formule -C₂H₄- ou -C₃H₆-, et
n et p signifiant chacun un nombre allant de 1 à 30, de préférence de 3 à 10,
ainsi que de l'eau en complément à 100 % en poids.

2. Dispersions d'aspect nacré selon la revendication 1, caractérisées en ce que, dans la formule I, R¹ signifie un alkyle en C₁₅-C₁₇, X signifie un groupe de formule R⁵-C(O)- avec R⁵ signifiant un même alkyle en C₁₅-C₁₇ et m vaut 1, dans la formule II, R² signifie un alkyle en C₁₀-C₁₈, R³ signifie un atome d'hydrogène, n va de 5 à 20 et p vaut 0.

3. Dispersions d'aspect nacré selon la revendication 1 ou 2, caractérisées en ce qu'elles contiennent de 15 à 30 % en poids de composés de formule I, de préférence de 20 à 25 % en poids, et de 5 à 15 % en poids de composés de formule II, par rapport au poids total.

4. Dispersions d'aspect nacré selon l'une des revendications 1 à 3, caractérisées en ce qu'elles contiennent de 5 à 40 % en poids d'alcools polyvalents de bas poids moléculaire et/ou de polyols, de préférence de 20 à 30 % en poids, par rapport au poids total.
